(19) Europäisches Patentamt
European Patent Office
Office européen des brevets

(11) Veröffentlichungsnummer: **0 229 660**
**B1**

(12) # EUROPÄISCHE PATENTSCHRIFT

(45) Veröffentlichungstag der Patentschrift: **26.09.90**

(21) Anmeldenummer: **87100347.1**

(22) Anmeldetag: **13.01.87**

(51) Int. Cl.[5]: **C07D 409/12**, A61K 31/44, A61K 31/47 // C07D333/32, C07D333/38

(54) **Neue Stickstoffarylmethoxy-thiophenderivate und deren Säureadditionssalze,Verfahren zu ihrer Herstellung und diese enthaltende pharmazeutische Präparate.**

(30) Priorität: **17.01.86 AT 107/86**

(43) Veröffentlichungstag der Anmeldung: **22.07.87 Patentblatt 87/30**

(45) Bekanntmachung des Hinweises auf die Patenterteilung: **26.09.90 Patentblatt 90/39**

(84) Benannte Vertragsstaaten: **AT BE CH DE ES FR GB GR IT LI LU NL SE**

(56) Entgegenhaltungen:
**EP-A- 0 050 957**
**GB-A- 2 065 121**
**GB-A- 2 068 950**
**US-A- 4 427 682**

(73) Patentinhaber: **CL PHARMA AKTIENGESELLSCHAFT, St. Peter-Strasse 25, A-4021 Linz(AT)**

(72) Erfinder: **Binder, Dieter, Dr., Sieveringerstrasse 207, A-1190 Wien(AT)**
Erfinder: **Rovenszky,Franz,Dr., Lagerhausstrasse 5/8, A-2460 Bruck a.d.Leitha(AT)**
Erfinder: **Ferber, Hubert Peter, Dr., Ahornweg 24, A-4052 Ansfelden(AT)**
Erfinder: **Schrör, Karsten, Dr., Hüttenweg 2, D-5020 Frechen-Königsdorf(DE)**

(74) Vertreter: **Kunz, Ekkehard, Dr., Chemie Holding AG Patentwesen St. Peter-Strasse 25, A-4021 Linz(AT)**

**EP 0 229 660 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Stickstoffarylmethoxy-thiophenderivate der allgemeinen Formel

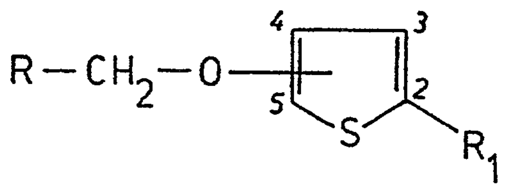

I

in der
die Gruppe $R-CH_2O-$ in Position 4 oder 5 des Thiophenrings steht,
R eine Gruppe 2-Pyridinyl oder 2-Chinolinyl und
R1 eine Gruppe $-COO-(C_1-C_4)$-Alkyl, $-CO-(CH_2)_n-CH_3$ oder $-CH(OH)-(CH_2)_n-CH_3$, wobei n eine ganze Zahl 2 bis 6 darstellt,
bedeuten,
sowie ihre Hydrate und/oder ihre pharmazeutisch verträglichen Säureadditionssalze, ein Verfahren zu ihrer Herstellung, pharmazeutische Präparate, die diese Verbindungen enthalten, sowie ihre Verwendung in Arzneimitteln.

Der Ausdruck $(C_1-C_4)$-Alkyl bezeichnet geradkettige oder verzweigte gesättigte Kohlenwasserstoffreste mit 1 bis 4 Kohlenstoffatomen, wie Methyl, Ethyl, Propyl, Isopropyl, Butyl, Isobutyl oder tert.Butyl.

In einer bevorzugten Klasse von Verbindungen der Formel I bedeutet $R_1$ eine Gruppe $-CO-(CH_2)_4-CH_3$, $-CH(OH)-(CH_2)_4-CH_3$ oder $-COOCH_3$.

Besonders bevorzugt sind die Verbindungen 1-(5-(2-Chinolinylmethoxy)-2-thienyl)-1-hexanon und Alpha-pentyl-5-(2-chinolinylmethoxy)-2-thiophenmethanol.

Die Stickstoffarylmethoxy-thiophenderivate der allgemeinen Formel I und deren Säureadditionssalze können dadurch hergestellt werden, daß man

a) eine Verbindung der Formel

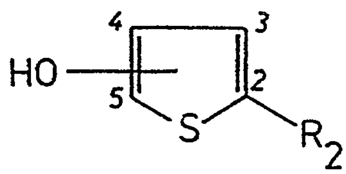

II

in der
die Hydroxylgruppe in Position 4 oder 5 des Thiophenringes steht und $R_2$ eine Gruppe $-COO-(C_1-C_4)$-Alkyl oder eine Gruppe $-CO-(CH_2)_n-CH_3$, wobei n eine ganze Zahl 2 bis 6 darstellt, bedeutet,
in Gegenwart von Alkalicarbonat in einem niedersiedenden polaren organischen Lösungsmittel mit einer Verbindung der Formel

$$R-CH_2-Cl \qquad III$$

in der
R die in Formel I angegebene Bedeutung besitzt, oder mit deren Hydrochlorid umsetzt und, falls $R_2$ eine Gruppe $-CO-(CH_2)_n-CH_3$ bedeutet, gegebenenfalls mit einem Reduktionsmittel zu einer Verbindung der Formel I, in der $R_1$ die Gruppe $-CH(OH)-(CH_2)_n-CH_3$ bedeutet, reduziert, oder

b) falls $R_1$ der Formel 1 den Rest $-CH(OH)-(CH_2)_n-CH_3$ oder $-CO(CH_2)_n-CH_3$ bedeutet,
eine Verbindung der Formel

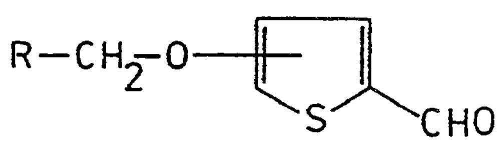

IV

in der
R die in Formel I angegebene Bedeutung besitzt,
mit einer Verbindung der Formel

$$CH_3-(CH_2)_n-Y \qquad V$$

in der

n eine ganze Zahl 2 bis 6 bedeutet und
Y die Gruppe Li oder Mg X, wobei
X Brom oder Jod darstellt, bedeutet, zu einer Verbindung der Formel I umsetzt, in der $R_1$ die Gruppe $-CH(OH)-(CH_2)_n-CH_3$ bedeutet, und gegebenenfalls die so erhaltene Verbindung mit einem Oxidationsmittel zu einer Verbindung der Formel I, in der $R_1$ die Gruppe $-CO-(CH_2)_n-CH_3$ bedeutet, oxidiert und

c) gegebenenfalls nach a) oder b) erhaltene Verbindungen der Formel I in ihre pharmazeutisch verträglichen Säureadditionssalze überführt.

Die Alkylierung von Verbindungen der Formel II mit den Halogenverbindungen der Formel III nach Weg a) kann in einem polaren, niedrig siedenden organischen Lösungsmittel, beispielsweise in Aceton oder Butanon, in Gegenwart von mindestens 1 Mol wasserfreiem Natrium- oder Kaliumcarbonat erfolgen. Bevorzugterweise wird die Reaktion in siedendem 2-Butanon durchgeführt. Bei Einsatz der Hydrochloride von Verbindungen der Formel III ist ein weiteres Mol Alkalicarbonat notwendig. Die Reaktionszeit beträgt ca. 5–12 Stunden, kann aber auch in Abhängigkeit von den Einsatzstoffen, den Lösungsmitteln und der Temperatur darüber oder darunter liegen.

Die fallweise Reduktion der so erhaltenen Verbindungen der Formel I, in der $R_1$ eine Gruppe $-CO-(CH_2)_n-CH_3$ bedeutet, zu Verbindungen der Formel I, in der $R_1$ eine Gruppe $-CH(OH)-(CH_2)_n-CH_3$ bedeutet, kann mit Reduktionsmitteln, die zur Reduktion einer Ketogruppe zur Alkoholgruppe geeignet sind, wie Lithiumaluminiumhydrid oder Natriumborhydrid, durchgeführt werden. Sie erfolgt beispielsweise in ca. 1 Stunde glatt, wenn man $NaBH_4$ in geringem molaren Überschuß in einem Alkohol mit 1–4 C-Atomen, bevorzugt in Ethanol als Lösungsmittel einsetzt und unter Rückfluß erhitzt.

Die Umsetzung von Verbindungen der Formel IV mit den metallorganischen Verbindungen der Formel V nach Weg b) kann nach den bei Grignardreaktionen üblichen Bedingungen erfolgen. Bewährt hat sich hiebei, die metallorganischen Verbindungen, gelöst in Ether oder THF, bei einer Temperatur von –20 bis +20°C, bevorzugt bei etwa 0°C, vorzulegen und die Lösung des Aldehyds der Formel IV in einem inerten organischen Lösungsmittel wie beispielsweise Ether oder Tetrahydrofuran, bevorzugt in THF, unter Kühlung zuzutropfen.

Die fallweise Oxidation der so erhaltnen Verbindungen der Formel I, in der $R_1$ eine Gruppe $-CH(OH)-(CH_2)_n-CH_3$ bedeutet, zu den Ketonen der Formel I kann mit üblichen Oxidationsmitteln wie Mangandioxid oder sechswertigten Chromverbindungen, bevorzugt mit verschiedenen sechswertigen Chromverbindungen erfolgen, beispielsweise mit Chromtrioxid in Eisessig oder Pyridin oder mit Pyridiniumchlorochromat in Methylenchlorid.

Die Verbindungen der Formel I haben schwachbasische Eigenschaften. Man kann sie daher auch mit entsprechenden starken Protonensäuren in kristalline pharmazeutisch verträgliche Säureadditionssalze überführen, die sich wie z.B. die Hydrochloride gut durch Umkristallisieren reinigen lassen. Dazu löst man die rohe Base in einem geeigneten Lösungsmittel, z.B. in einem niederen Alkohol, gibt eine mindestens äquivalente Menge starker Protonensäure dazu, dampft im Vakuum das Lösungsmittel ab und kristallisiert den Rückstand beispielsweise aus Methanol oder Ethanol, gegebenenfalls unter Zusatz von Ether, um.

Beispiele für derartige pharmazeutisch verträgliche Säureadditionssalze sind neben dem Salz der Salzsäure das der Schwefelsäure, Salpetersäure, Phosphorsäure sowie Additionssalze mit organischen Säuren wie Essigsäure, Zitronensäure, Oxalsäure, Malonsäure, Salicylsäure, Methansulfonsäure u.ä. Diese Säureadditionssalze besitzen eine ebensogroße pharmakologische Wirkung wie die entsprechenden freien Basen der Formel I.

Die Verbindungen der Formel III und V sind literaturbekannt. Die Verbindungen der Formeln II und IV können, ausgehend von bekannten Produkten, in an sich bekannter Weise hergestellt werden.

Die Verbindungen der Formel IV können aus Verbindungen der Formel I, in der $R_1$ eine Gruppe $-COO-(C_1-C_4)$-Alkyl bedeutet, hergestellt werden. Hiezu reduziert man diese Verbindungen, beispielsweise mit Lithiumaluminiumhydrid in THF unter Kühlen auf eine Temperatur von unter 10°C.

Die Oxidation der so erhaltenen Alkohole zu den gewünschten Aldehyden wird vorzugsweise mit überschüssigem Pyridiniumchlorochromat in Methylenchlorid bei Raumtemperatur durchgeführt.

Die Verbindungen der Formel II können insbesondere gemäß dem folgenden Reaktionsschema und den spezifischen Angaben in den Beispielen synthetisiert werden.

Reaktionsschema

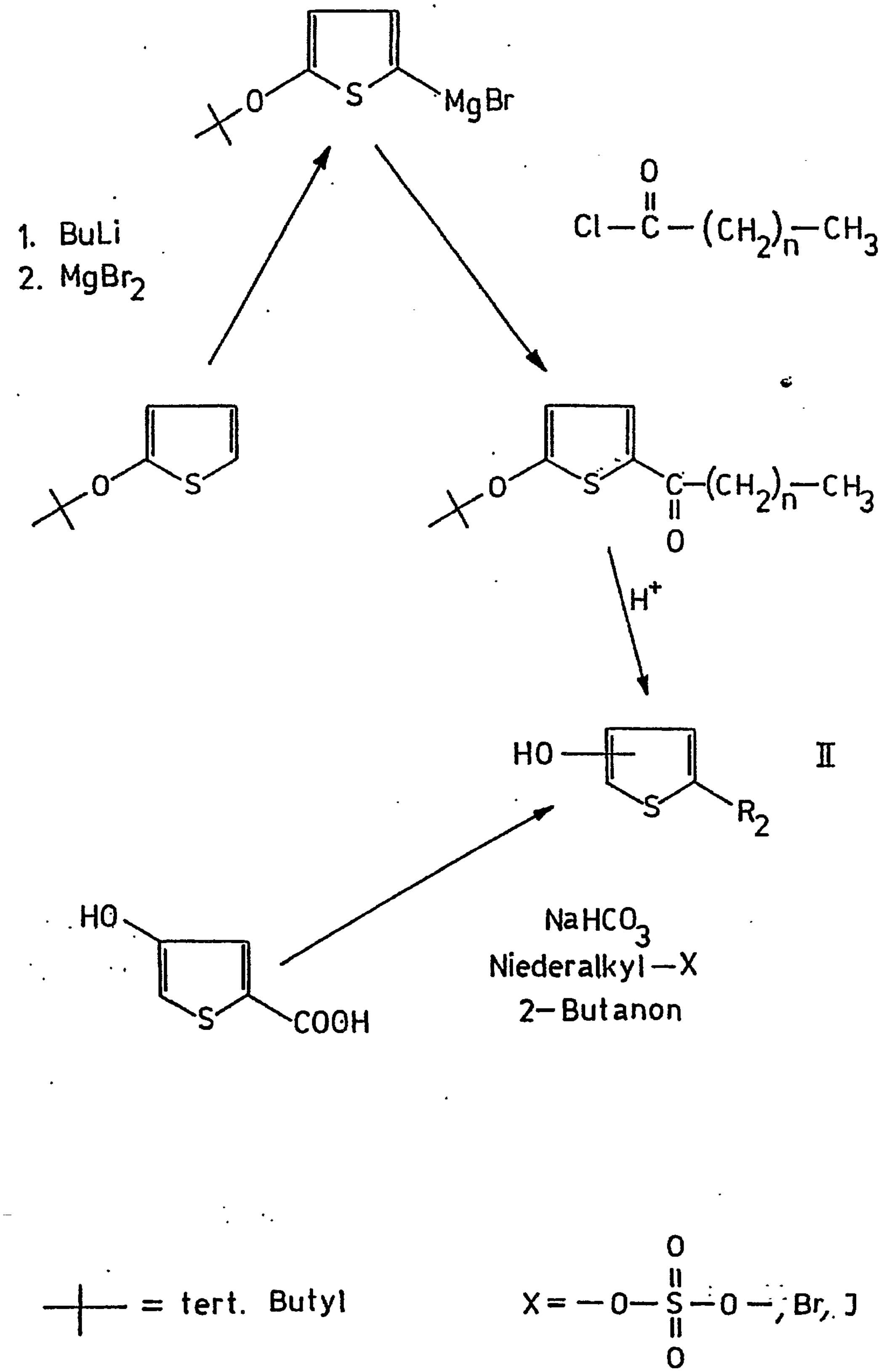

Die Verbindungen der Formel I und ihre pharmazeutisch verwendbaren Säureadditionssalze besitzen wertvolle pharmakologische Eigenschaften. Insbesondere besitzen sie eine spezifische Hemmwirkung auf bestimmte Enzyme, deren Substrat die Arachidonsäure ist. Diese Enzyme regeln in Enzymkaskaden die Biosynthese der Prostaglandine (PG), des Thromboxan $A_2$ (TX $A_2$) und der Leukotriene (LT).

Vor allem die 5-Lipoxygenase, die die Arachidonsäure in die 5-Hydroperoxyeikosatetraensäure – einer Vorstufe der Leukotriene – umwandelt, wird von den Verbindungen der Formel I spezifisch gehemmt. Daneben hemmen die Verbindungen der Formel I, in denen $R_1$ den Rest –CO-niederalkyl bedeutet, auch die Cyclooxygenase, ein Enzym, das die Arachidonsäure in das Prostaglandin $G_2$ – einer Vorstufe für andere Prostaglandine und des $TXA_2$ – verwandelt.

Aufgrund dieser wertvollen pharmakologischen Eigenschaften können die Verbindungen der Formel I sowie ihre pharmazeutisch verträglichen Additionssalze in der Humanmedizin bei Erkrankungen, die durch eine Störung des Prostaglandin-, Thromboxan $A_2$ – oder Leukotrienstoffwechsels hervorgerufen werden, verwendet werden. Bei solchen Erkrankungen handelt es sich beispielsweise um rheumatische Arthritis, allergische Erkrankungen und Asthma.

Zur Untersuchung der pharmakologischen Eigenschaften der erfindungsgemäßen Substanzen wurde deren Hemmwirkung auf die durch den Kalzium-Ionophor A 23–187 induzierte Leukotrien $B_4$–($LTB_4$) Bildung gemessen. Hiezu wurden Human-Granulozyten gewaschen, die Zellzahl auf $10^7$ Zellen pro ml eingestellt und die Zellen für 10 Minuten bei 37°C mit den erfindungsgemäßen Verbindungen, wie zum Beispiel 1-(5-(2-Chinolinylmethoxy)-2-thienyl)-1-hexanon (Verbindung nach Beispiel 3) oder alpha-Pentyl-5-(2-Chinolinylmethoxy)-2-thiophenmethanol (Verbindung gemäß Beispiel 5) die in DMSO auf $10^{-2}$ M gelöst und mit physiologischer NaCl auf die gewünschte Konzentration verdünnt worden waren, inkubiert. Anschließend wurden die Zellen nach Zusatz von A 23 187 (Endkonzentration 1 µM) weitere 10 Minuten inkubiert. Die Reaktion wurde durch Zusatz von Eisessig gestoppt. Es wurde zentrifugiert, der Überstand abpipettiert und Prostaglandin $B_2$ als interner Standard zugesetzt.

Die Lipidfraktion wurde über HPLC aufgetrennt, wobei $LTB_4$ sowie seine 20-OH- und 20-COOH-Metabolite bei 280 nm photometrisch bestimmt wurden.

Für jeden Konzentrationswert der erfindungsgemäßen Substanzen wurden 2 Versuche durchgeführt und für jede Substanz die Konzentration, die zu einer 50%-igen Hemmung ($IC_{50}$) der $LTB_4$-Bildung führte, ermittelt. Hiebei betrug beispielsweise die $IC_{50}$ 0,2 µM für die Verbindung gemäß Beispiel 3 und 1,0 µM für die Verbindung gemäß Beispiel 5.

Die Verbindungen der allgemeinen Formel I sowie deren Salze können als Heilmittel, z.B. in Form pharmazeutischer Präparate Verwendung finden, welche die erfindungsgemäßen Verbindungen in Mischung mit einem für die enterale oder parenterale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen enthalten. Die pharmazeutischen Präparate können in fester Form z.B. als Tabletten, Dragees, Suppositorien, Kapseln, oder in flüssiger Form z.B. als Lösungen, Suspensionen oder Emulsionen vorliegen. Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer. Sie können auch in Kombination mit anderne therapeutisch wertvollen Stoffen verabreicht werden.

Beispiel 1:

5-(2-Chinolinylmethoxy)-2-thiophencarbonsäuremethylester

8,0 g (50,6 mmol) 5-Hydroxy-2-thiophencarbonsäuremethylester, 9,0 g (50,6 mmol) 2-Chlormethylchinolin und 7,0 g (50,6 mmol) Kaliumcarbonat werden unter Stickstoff in 100 ml abs. 2-Butanon 8 Stunden unter Rückfluß erhitzt. Anschließend wird eingedampft und der Rückstand zwischen gesättigter Natriumbicarbonat-Lösung und Methylenchlorid verteilt. Die wäßrige Phase wird noch zweimal mit Methylenchlorid extrahiert, über Natriumsulfat unter Zusatz von Aktivkohle getrocknet, filtriert und eingedampft. Das Rohprodukt (14 g braunes Öl) wird in wenig Methanol gelöst und mit überschüssiger methanolischer Salzsäure versetzt. Es wird eingedampft und der Rückstand aus Methanol umkristallisiert.

Die so erhaltenen Kristalle werden zwischen gesättigter Natriumbicarbonat-Lösung und Methylenchlorid verteilt, die wäßrige Phase noch zweimal mit Methylenchlorid extrahiert, die vereinigten organischen Phasen über Natriumsulfat getrocknet, filtriert und eingedampft. Der Rückstand wird zweimal aus Ethanol umkristallisiert.
Ausbeute: 3,9 g farblose Kristalle (26%)
Schmp: 82–3°C (Ethanol)

Beispiel 2:

5-(2-Chinolinylmethoxy)-2-thiophencarbonsäuremethylester, Hydrochlorid

1.0 g (3,34 mmol) 5-(2-Chinolinylmethoxy)-2-thiophencarbonsäuremethylester wird in Methanol gelöst,

mit 3,5 ml 1n methanolischer Salzsäure versetzt und die Lösung bis zur Trockene eingedampft. Der Rückstand wird aus Methanol umkristallisiert.
Ausbeute: 0,88 g farblose Kristalle (78%)
Schmp.: 136–9°C (Methanol)

Beispiel 3:

1-(5-(2-Chinolinylmethoxy)-2-thienyl)-1-hexanon

20,0 g (0,101 mol) 1-(5-Hydroxy-2-thienyl)-1-hexanon werden mit 13,9 g (0,101 mol) Kaliumcarbonat und 17,9 g (0,101 mol) 2-Chlormethylchinolin in 220 ml abs. 2-Butanon 8 Stunden unter Stickstoff unter Rückfluß erhitzt.

Das Lösungsmittel wird im Vakuum abdestilliert und der Rückstand zwischen Ether und gesättigter Natriumbicarbonatlösung verteilt. Die wäßrige Phase wird noch dreimal mit je 200 ml Ether extrahiert. Die vereinigten organischen Phasen werden einmal mit Wasser gewaschen, über Natriumsulfat/Aktivkohle getrocknet, filtriert und eingedampft.

Das Rohprodukt (20,0 g, 58%) wird mit Diisopropylether digeriert und aus Aceton umkristallisiert.
Ausbeute: 12,2 g farblose Kristalle (36%)
Schmp.: 86–8°C (Aceton)

Das Ausgangsmaterial kann wie folgt hergestellt werden:

1-(5-(1,1-Dimethyl-ethoxy)-2-thienyl)-1-hexanon

96,0 g (0,614 mol) 2-(1,1-Dimethyl-ethoxy)-thiophen werden in 600 ml abs. Ether gelöst und bei einer Temperatur unter 5°C 280 ml 2,5 molare Lösung von n-Butyllithium in n-Hexan (0,700 mol) innerhalb von 30 Minuten zugetropft. Es wird auf Raumtemperatur erwärmen gelassen und noch 2 Stunden unter Rückfluß erhitzt.

Anschließend wird abgekühlt und Magnesiumbromid in Ether (hergestellt aus 117,9 g (0,738 mol) Brom und 26,8 g (1,102 gr.at) Magnesium in 500 ml abs. Ether) bei einer Temperatur unter 10°C zugetropft. Es wird noch 1 Stunde bei Raumtemperatur gerührt.

Die oben hergestellte Lösung wird bei 17°C innerhalb von 2 Stunden zu einer Lösung von 82,7 g (0,614 mol) Capronsäurechlorid in 250 ml abs. Ether zugetropft. Es wird noch eine halbe Stunde bei Raumtemperatur gerührt und auf 1 l gesättigte Natriumbicarbonatlösung geleert, wobei ein dicker Niederschlag entsteht. Die organische Phase wird abdekantiert und der Rückstand gut mit Ether gewaschen. Die vereinigten organischen Phasen werden mit Wasser gewaschen, über Natriumsulfat/Aktivkohle getrocknet, filtriert und eingedampft. Der Rückstand (141,8 g rotes Öl, 91%) wird im Feinvakuum destilliert.
Ausbeute: 59,2 g hellgelbes Öl (38%)
Kp.: 120–130°C/0,12–0,16 mbar.

1-(5-Hydroxy-2-thienyl)-1-hexanon

55,0 g (0,216 mol) 1-(5-(1,1-Dimethyl-ethoxy)-2-thienyl)-1-hexanon werden in 400 ml Methanol und 24 ml konz. Salzsäure 2,5 Stunden unter Rückfluß erhitzt. Anschließend wird im Vakuum eingedampft, der Rückstand in 500 ml Ether aufgenommen und zweimal mit je 50 ml Wasser gewaschen. Die organische Phase wird über Natriumsulfat getrocknet, filtriert und eingedampft. Der Rückstand (42,4 g dunkelbraune Kristalle, 99%) wird mit Petrolether : Diisopropylether = 7:3 digeriert.
Ausbeute: 27,0 g beige Kristalle (63%)
Schmp.: 70–1°C (Diisopropylether/Petrolether)

Beispiel 4:

1-(5-(2-Pyridinylmethoxy)-2-thienyl)-1-hexanon

1,0 g (5,04 mmol) 1-(5-Hydroxy-2-thienyl)-1-hexanon wird mit 0,75 g (4,60 mmol) 2-Chlormethylpyridin, Hydrochlorid und 1,4 g (10,1 mmol) Kaliumcarbonat in 20 ml abs. 2-Butanon unter Stickstoff 12 Stunden unter Rückfluß erhitzt. Anschließend wird im Vakuum eingedampft und der Rückstand zwischen gesättigter Natriumcarbonatlösung und Ether verteilt. Es wird noch mehrmals mit Ether extrahiert, die vereinigten organischen Phasen über Natriumsulfat/Aktivkohle getrocknet, filtriert und eingedampft. Der Rückstand wird mit Diisopropylether digeriert.
Ausbeute: 0,76 g farblose Kristalle (57%)
Schmp.: 58–9°C.

Beispiel 5:

Alpha-pentyl-5-(2-chinolinylmethoxy)-2-thiophenmethanol

Zu 7,5 g (0,022 mol) 1-(5-(2-Chinolinylmethoxy)-2-thienyl)-1-hexanon in 100 ml Ethanol wird 1,0 g (0,027 mol) $NaBH_4$ zugegeben und 40 Minuten unter Rückfluß erhitzt. Das Reaktionsgemisch wird mit Wasser verdünnt und dreimal mit je 50 ml Methylenchlorid extrahiert. Die vereinigten organischen Phasen werden über Natriumsulfat getrocknet, filtriert und eingedampft. Der Rückstand wird aus Ethanol umkristallisiert.
Ausbeute: 6,5 g farblose Kristalle (86%)
Schmp.: 87–90°C (Ethanol)

Beispiel 6:

4-(2-Chinolinylmethoxy)-2-thiophencarbonsäuremethylester

4,70 g (29,7 mmol) 4-Hydroxy-2-thiophencarbonsäuremethylester, 5,28 g (29,7 mmol) 2-Chlormethylchinolin und 4,11 g (29,7 mmol) Kaliumcarbonat werden unter Stickstoff in 150 ml abs. 2-Butanon 10 Stunden unter Rückfluß erhitzt. Anschließend wird eingedampft und der Rückstand zwischen gesättigter Natriumbicarbonat-Lösung und Methylenchlorid verteilt. Die wäßrige Phase wird noch dreimal mit je 80 ml Methylenchlorid extrahiert, die vereinigten organischen Phasen über Natriumsulfat/Aktivkohle getrocknet, filtriert und eingedampft. Der Rückstand (8,87 g rötliche Kristalle, 100%) wird aus Methanol umkristallisiert.
Ausbeute: 4,98 g farblose Kristalle (56%)
Schmp.: 123–4°C (Methanol)

Das Ausgangsmaterial kann wie folgt hergestellt werden:

4-Hydroxy-2-thiophencarbonsäuremethylester

50,0 g (0,347 mol) 4-Hydroxy-2-thiophencarbonsäure und 58,3 g (0,694 mol) Natriumbicarbonat werden in 900 ml abs. 2-Butanon unter Stickstoff zum Sieden erhitzt und 43,7 g (0,347 mol) Dimethylsulfat innerhalb von 20 Minuten zugetropft. Es wird noch 2,5 Stunden unter Rückfluß erhitzt. Anschließend wird im Vakuum eingedampft und der Rückstand zwischen gesättigter Natriumcarbonatlösung und Ether verteilt. Die wäßrige Phase wird noch fünfmal mit je 80 ml Ether extrahiert. Die vereinigten organischen Phasen werden über Natriumsulfat/Aktivkohle getrocknet, filtriert und eingedampft.
Ausbeute: 49,6 g gelbliche Kristalle (90%)
Schmp.: 84–5°C (Diisopropylether/Petrolether)

Beispiel 7:

4-(2-Chinolinylmethoxy)-2-thiophencarbonsäuremethylester, Hydrochlorid

1,0 g (3,34 mmol) 4-(2-Chinolinylmethoxy)-2-thiophencarbonsäuremethylester wird in 50 ml Methanol unter gelindem Erwärmen gelöst, 4 ml 1n methanolische Salzsäure zugesetzt und die Lösung im Vakuum zur Trockene eingedampft. Der Rückstand wird aus Methanol umkristallisiert.
Ausbeute: 1,03 g farblose Kristalle (92%)
Schmp.: 162–5°C (Zers. ab 155°C, Methanol)

Beispiel 8:

4-(2-Pyridinylmethoxy)-2-thiophencarbonsäuremethylester

3,00 g (19,0 mmol) 4-Hydroxy-2-thiophencarbonsäuremethylester, 3,11 g (19,0 mmol) 2-Chlormethylpyridin, Hydrochlorid und 5,24 g (37,9 mmol) Kaliumcarbonat werden unter Stickstoff in 60 ml abs. 2-Butanon 10 Stunden unter Rückfluß erhitzt. Anschließend wird im Vakuum eingedampft und der Rückstand zwischen Methylenchlorid und gesättigter Natriumbicarbonat-Lösung verteilt. Die wäßrige Phase wird noch zweimal mit je 100 ml Methylenchlorid extrahiert, die vereinigten organischen Phasen über Natriumsulfat/Aktivkohle getrocknet, filtriert und eingedampft. Der Rückstand wird mit Diisopropylether digeriert.
Ausbeute: 3,12 g farblose Kristalle (66%)
Schmp.: 65–6°C

Beispiel 9:

Alpha-pentyl-4-(2-chinolinmethoxy)-2-thiophenmethanol

Zu 1,46 g (0,0601 gr.at) Magnesium in 50 ml abs. Ether werden 8,20 g (0,0543 mol) 1-Brompentan, gelöst in 20 ml Ether, so zugetropft, daß leichter Rückfluß erhalten bleibt. Nach beendeter Zugabe wird noch 30 Minuten unter Rückfluß erhitzt. Anschließend wird auf –5°C gekühlt und unter trockenem Stickstoff 7,31 g (27,1 mmol) 4-(2-Chinolinylmethoxy)-2-thiophenaldehyd, gelöst in 60 ml abs. THF, so zugetropft, daß die Temperatur 0°C nicht übersteigt. Es wird noch 30 Minuten bei 0°C gerührt und anschließend das Reaktionsgemisch auf 100 ml gesättigte Natriumcarbonat-Lösung und 100 g Eis gegossen. Der ausgefallene Niederschlag wird über Hyflo abgesaugt, die Phasen getrennt und die wäßrige Phase noch dreimal mit je 100 ml Ether extrahiert. Die vereinigten organischen Phasen werden über Natriumsulfat/Aktivkohle getrocknet, filtriert und eingedampft. Der ölige Rückstand wird mit Diisopropylether kristallisiert und aus Diisopropylether umkristallisiert.
Ausbeute: 5,26 g hellgelbe Kristalle (57%)
Schmp.: 46–8°C (Diisopropylether)

Das Ausgangsmaterial kann wie folgt hergestellt werden:

4-(2-Chinolinylmethoxy)-2-thiophenmethanol

25,0 g (83,5 mmol) 4-(2-Chinolinylmethoxy)-2-thiophencarbonsäuremethylester werden in 500 ml abs. THF gelöst und im Stickstoffgegenstrom unter Kühlen 2,54 g (66,8 mmol) $LiAlH_4$ portionsweise so zugegeben, daß die Temperatur 5°C nicht übersteigt. Es wird noch 10 Minuten bei 5°C gerührt und anschließend tropfenweise 10 ml Eiswasser zugegeben. Es wird über Hyflo abgesaugt und der Hydroxidniederschlag mehrmals mit Ether gewaschen. Die Phasen werden getrennt und die wäßrige mit Natriumchlorid gesättigt. Es wird noch dreimal mit Ether extrahiert, die vereinigten organischen Phasen über Natriumsulfat/Aktivkohle getrocknet, filtriert und eingedampft.
Ausbeute: 21,0 g farblose Kristalle (93%)
Schmp.: 126–8°C (Methanol)

4-(2-Chinolinylmethoxy)-2-thiophenaldehyd

11,7 g (54,2 mmol) Pyridiniumchlorochromat werden in 60 ml abs. Methylenchlorid suspendiert und unter heftigem Rühren 9,8 g (36,1 mmol) 4-(2-Chinolinylmethoxy)-2-thiophenmethanol in 110 ml abs. Methylenchlorid bei Raumtemperatur auf einmal zugegeben. Es wird noch 1,5 Stunden bei Raumtemperatur gerührt, über Hyflo abgesaugt und der Rückstand viermal mit trockenem Ether gewaschen. Das Filtrat wird mit Aktivkohle versetzt, filtriert und eingedampft. Der Rückstand wird mehrmals mit insgesamt 400 ml Diisopropylether ausgekocht, die Lösung mit Aktivkohle filtriert, auf 80 ml eingeengt und auf –20°C gekühlt. Die ausgefallenen Kristalle werden abgesaugt und mit wenig kaltem Diisopropylether gewaschen.
Ausbeute: 4,67 g gelbliche Kristalle (48%)
Schmp.: 88–91°C (Diisopropylether)

Beispiel 10:

1-(4-(2-Chinolinylmethoxy)-2-thienyl)-1-hexanon

2,20 g (6,44 mmol) Alpha-pentyl-4-(2-chinolinylmethoxy)-2-thiophenmethanol werden in 10 ml Eisessig gelöst und unter Kühlen eine Lösung von 1,40 g (14,0 mmol) $CrO_3$ in 30 ml Eisessig bei 20°C zugetropft. Es wird noch 30 Minuten gerührt und das Reaktionsgemisch im Vakuum eingedampft. Der Rückstand wird mit 50 ml Wasser versetzt und mit Kaliumcarbonat neutralisiert. Es wird dreimal mit je 30 ml Ether extrahiert. Die vereinigten organischen Phasen werden einmal mit Wasser gewaschen, über Natriumsulfat/Aktivkohle getrocknet und eingedampft. Der ölige Rückstand (1,40 g, 64%) wird mit Methanol kristallisiert und zweimal aus Methanol umkristallisiert.
Ausbeute: 0,78 g gelbliche Kristalle (36%)
Schmp.: 53–5°C (Methanol)

**Patentansprüche für die Vertragsstaaten: BE, CH, FR, GB, IT, LI, LU, NL, SE**

1. Neue Stickstoffarylmethoxy-thiophenderivate der allgemeinen Formel

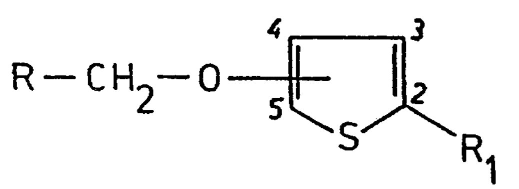

I

in der
die Gruppe $R-CH_2O-$ in Position 4 oder 5 des Thiophenrings steht,
R eine Gruppe 2-Pyridinyl oder 2 Chinolinyl und
$R_1$ eine Gruppe $-COO-(C_1-C_4)$-Alkyl, $-CO-(CH_2)_n-CH_3$ oder $-CH(OH)-(CH_2)_n-CH_3$, wobei n eine ganze Zahl 2 bis 6 darstellt,
bedeuten,
sowie ihre Hydrate und/oder ihre pharmazeutisch verträglichen Säureadditionssalze.

2. Verbindungen der allgemeinen Formel I nach Anspruch 1, worin $R_1$ eine Gruppe $-CO-(CH_2)_4-CH_3$, $-CH(OH)-(CH_2)_4-CH_3$ oder $-COOCH_3$ bedeutet.

3. Die Verbindung der allgemeinen Formel I nach Anspruch 1 oder 2 1-(5-(2-Chinolinylmethoxy)-2-thienyl)-1-hexanon.

4. Die Verbindung der allgemeine Formel I nach Anspruch 1 oder 2 Alpha-pentyl-5-(2-chinolinylmethoxy-)2-thiophenmethanol.

5. Verfahren zur Herstellung von Verbindungen der im Anspruch 1 definierten allgemeinen Formel I, dadurch gekennzeichnet, daß man

a) eine Verbindung der allgemeinen Formel

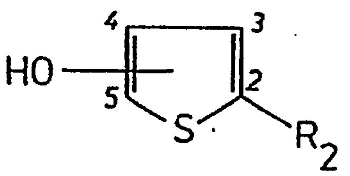

II

in der
die Hydroxylgruppe in Position 4 oder 5 des Thiophenringes steht und
$R_2$ eine Gruppe $-COO-(C_1-C_4)$-Alkyl oder eine Gruppe $-CO-(CH_2)_n-CH_3$, wobei n eine ganze Zahl 2 bis 6 darstellt, bedeutet,
in Gegenwart von Alkalicarbonat in einem niedersiedenden polaren organischen Lösungsmittel mit einer Verbindung der Formel

$$R-CH_2-Cl \qquad III$$

in der
R die in Formel I angegebene Bedeutung besitzt, oder mit deren Hydrochlorid, umsetzt und, falls $R_2$ eine Gruppe $-CO-(CH_2)_n-CH_3$ bedeutet, gegebenenfalls mit einem Reduktionsmittel zu einer Verbindung der Formel I, in der $R_1$ die Gruppe $-CH(OH)-(CH_2)_n-CH_3$ bedeutet, reduziert, oder

b) falls $R_1$ der Formel I den Rest $-CH(OH)-(CH_2)_n-CH_3$ oder $-CO-(CH_2)_n-CH_3$ bedeutet,
eine Verbindung der Formel

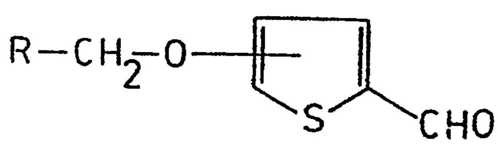

IV

in der
R die in Formel I angegebene Bedeutung besitzt, mit einer Verbindung der Formel

$$CH_3-(CH_2)_n-Y \qquad V$$

in der
n eine ganze Zahl 2 bis 6 bedeutet und
Y die Gruppe Li oder Mg X, wobei
X Brom oder Jod darstellt, bedeutet, zu einer Verbindung der Formel I umsetzt, in der $R_1$ die Gruppe $-CH(OH)-(CH_2)n-CH_3$ bedeutet, und gegebenenfalls die so erhaltene Verbindung mit einem Oxidationsmittel zu einer Verbindung der Formel I, in der $R_1$ die Gruppe $-CO-(CH_2)_n-CH_3$ bedeutet, oxidiert und

c) gegebenenfalls nach a) oder b) erhaltene Verbindungen der Formel I in ihre pharmazeutisch verträglichen Säureadditionssalze überführt.

6. Verfahren nach Anspruch 5 zur Herstellung von Verbindungen der allgemeinen Formel I, in der $R_1$ eine Gruppe $-CH(OH)-(CH_2)_n-CH_3$ bedeutet, dadurch gekennzeichnet, daß man zur Reduktion der Verbindungen der Formel I, in der $R_1$ eine Gruppe $-CO-(CH_2)_n-CH_3$ bedeutet, Natriumborhydrid in Ethanol verwendet.

7. Verfahren nach Anspruch 5 zur Herstellung von Verbindungen der allgemeinen Formel I, in der $R_1$ eine Gruppe $-CH(OH)-(CH_2)_n-CH_3$ oder $-CO-(CH_2)_n-CH_3$ bedeutet, dadurch gekennzeichnet, daß

man Verbindungen der Formel IV mit Lithiumalkylen oder Grignard-Verbindungen in Ether oder THF zu Verbindungen der Formel I, in der $R_1$ eine Gruppe $-CH(OH)-(CH_2)_n-CH_3$ bedeutet, umsetzt und diese gegebenenfalls mit sechswertigen Chromverbindungen zu Verbindungen der Formel I des Formelblattes, in der $R_1$ eine Gruppe $-CO-(CH_2)_n-CH_3$ bedeutet, oxidiert.

8. Pharmazeutische Präparate, enthaltend Verbindungen der allgemeinen Formel I nach Anspruch 1 in Kombination mit üblichen galenischen Hilfs- und/oder Trägerstoffen.

9. Verbindungen nach Anspruch 1 zur Verwendung als Wirkstoffe für Arzneimittel zur Behandlung und Prophylaxe von Erkrankungen, die durch Störungen des Arachidonsäurestoffwechsels hervorgerufen werden.

**Patentansprüche für die Vertragsstaaten: AT, GR, ES**

1. Verfahren zur Herstellung neuer Stickstoffarylmethoxy-thiophenderivate der allgemeinen Formel

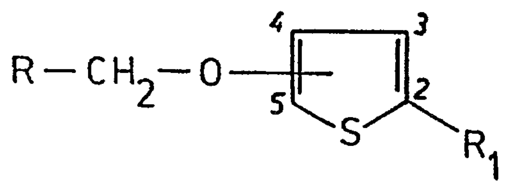

I

in der
die Gruppe $R-CH_2O-$ in Position 4 oder 5 des Thiophenringes steht,
R eine Gruppe 2-Pyridinyl oder 2 Chinolinyl und
$R_1$ eine Gruppe $-COO-(C_1-C_4)$-Alkyl, $-CO-(CH_2)_n-CH_3$ oder $-CH(OH)-(CH_2)_n-CH_3$, wobei n eine ganze Zahl 2 bis 6 darstellt,
bedeuten,
sowie ihrer Hydrate und/oder ihrer pharmazeutisch verträglichen Säureadditionssalze, dadurch gekennzeichnet, daß man

a) eine Verbindung der allgemeinen Formel

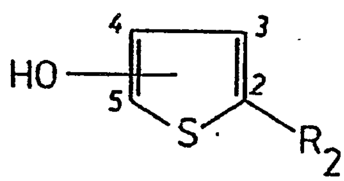

II

in der
die Hydroxylgruppe in Position 4 oder 5 des Thiophenringes steht und
$R_2$ eine Gruppe $-COO-(C_1-C_4)$-Alkyl oder eine Gruppe $-CO-(CH_2)_n-CH_3$, wobei n eine ganze Zahl 2 bis 6 darstellt, bedeutet, in Gegenwart von Alkalicarbonat in einem niedersiedenden polaren organischen Lösungsmittel mit einer Verbindung der Formel

$R-CH_2-Cl$ III

in der
R die in Formel I angegebene Bedeutung besitzt, oder mit deren Hydrochlorid, umsetzt und, falls $R_2$ eine Gruppe $-CO-(CH_2)_n-CH_3$ bedeutet, gegebenenfalls mit einem Reduktionsmittel zu einer Verbindung der Formel I, in der $R_1$ die Gruppe $-CH(OH)-(CH_2)_n-CH_3$ bedeutet, reduziert, oder

b) falls $R_1$ der Formel I den Rest $-CH(OH)-(CH_3)_n-CH_3$ oder $-CO-(CH_2)_n-CH_3$ bedeutet, eine Verbindung der Formel

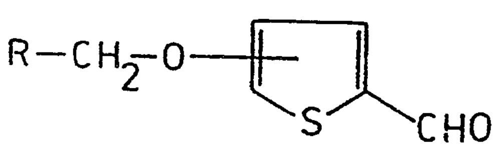

IV

in der
R die in Formel I angegebene Bedeutung besitzt, mit einer Verbindung der Formel

$CH_3-(CH_2)_n-Y$ V

in der
n eine ganze Zahl 2 bis 6 bedeutet und
Y die Gruppe Li oder Mg X, wobei
X Brom oder Jod darstellt, bedeutet, zu einer Verbindung der Formel I umsetzt, in der $R_1$ die Gruppe $-CH(OH)-(CH_2)_n-CH_3$ bedeutet, und gegebenenfalls die so erhaltene Verbindung mit einem Oxidationsmittel zu einer Verbindung der Formel I, in der $R_1$ die Gruppe $-CO-(CH_2)_n-CH_3$ bedeutet, oxidiert und
c) gegebenenfalls nach a) oder b) erhaltene Verbindungen der Formel I in ihre pharmazeutisch verträglichen Säureadditionssalze überführt.

2. Verfahren zur Herstellung von Verbindungen der allgemeinen Formel I nach Anspruch 1, worin $R_1$ eine Gruppe $-CO-(CH_2)_4-CH_3$ oder $-COOCH_3$ bedeutet, dadurch gekennzeichnet, daß man Verbindungen der Formel II, in der $R_2$ eine Gruppe $-CO-(CH_2)_4$ oder $-COOCH_3$ bedeutet, mit Verbindungen der Formel III umsetzt.

3. Verfahren zur Herstellung von Verbindungen der allgemeinen Formel I nach Anspruch 1, worin $R_1$ eine Gruppe $-CH(OH)-(CH_2)_4-CH_3$ bedeutet, dadurch gekennzeichnet, daß man Verbindungen der Formel II, in der $R_2$ eine Gruppe $-CO-(CH_2)_4-CH_3$ bedeutet, mit Verbindungen der Formel III umsetzt und anschließend zu Verbindungen der Formel I, in der $R_1$ eine Gruppe $-CH(OH)-(CH_2)_4-CH_3$ bedeutet, reduziert.

4. Verfahren nach einem der Ansprüche 1 oder 2, dadurch gekennzeichnet, daß man 1-(5-Hydroxy-2-thienyl)-1-hexanon mit 2-Chlormethylchinolin umsetzt.

5. Verfahren nach Anspruch 1 oder 3, dadurch gekennzeichnet, daß man 1-(5-Hydroxy-2-thienyl)-1-hexanon mit 2-Chlormethylchinolin umsetzt und anschließend die Ketogruppe zum sekundären Alkohol reduziert.

6. Verfahren nach einem der Ansprüche 1 bis 3 zur Herstellung von Verbindungen der allgemeinen Formel I, in der $R_1$ eine Gruppe $-CH(OH)-(CH_2)_n-CH_3$ bedeutet, dadurch gekennzeichnet, daß man die Umsetzung der Verbindungen der Formel II mit Verbindungen der Formel III in siedendem 2-Butanon in Gegenwart vcn mindestens 1 Mol wasserfreiem Natrium- oder Kaliumcarbonat durchführt.

7. Verfahren nach einem der Ansprüche 1 oder 3 zur Herstellung von Verbindungen der allgemeinen Formel I, in der $R_1$ eine Gruppe $-CH(OH)-(CH_2)_n-CH_3$ bedeutet, dadurch gekennzeichnet, daß man zur Reduktion der Verbindungen der Formel I, in der $R_1$ eine Gruppe $-CO-(CH_2)_n-CH_3$ bedeutet, Lithiumaluminiumhydrid oder Natriumborhydrid verwendet.

8. Verfahren nach Anspruch 1 zur Herstellung von Verbindungen der allgemeinen Formel I, in der $R_1$ eine Gruppe $-CH(OH)-(CH_2)_n-CH_3$ oder $-CO-(CH_2)_n-CH_3$ bedeutet, dadurch gekennzeichnet, daß man Verbindungen der Formel IV mit Lithiumalkylen oder Grignard-Verbindungen in Ether oder THF zu Verbindungen der Formel I des Formelblattes, in der $R_1$ eine Gruppe $-CH(OH)-(CH_2)_n-CH_3$ bedeutet, umsetzt und diese gegebenenfalls mit sechswertigen Chromverbindungen zu Verbindungen der Formel I des Formelblattes, in der $R_1$ eine Gruppe $-CO(CH_2)_n-CH_3$ bedeutet, oxidiert.

9. Verfahren zur Herstellung von pharmazeutischen Präparaten, dadurch gekennzeichnet, daß man Verbindungen der allgemeinen Formel I mit für die orale oder parenterale Applikation geeigneten pharmazeutischen, organischen oder anorganischen Hilfs- und/oder Trägerstoffen und gegebenenfalls anderen pharmazeutischen Wirkstoffen zu festen, halbfesten oder flüssigen Arzneiformen verarbeitet.

**Claims for the Contracting States: BE, CH, FR, GB, IT, LI, LU, NL, SE**

1. New nitrogen-arylmethoxy-thiophene derivatives of the general formula

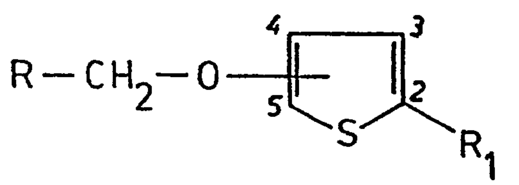

I

in which the $R-CH_2O-$ group is in position 4 or 5 of the thiophene ring, R denotes a 2-pyridinyl or 2-quinolinyl group and $R_1$ denotes a $-COO-(C_1-C_4)$-alkyl, $-CO-(CH_2)_n-CH_3$ or $CH(OH)-CH_2)_n-CH_3$ group, wherein n represents an integer from 2 to 6, and their hydrates and/or their pharmaceutically acceptable acid addition salts.

2. Compounds of the general formula I according to Claim 1, wherein $R_1$ denotes a $-CO-(CH_2)_4-CH_3$, $-CH(OH)-(CH_2)_4-CH_3$ or $-COOCH_3$ group.

3. Compound of the general formula I according to Claim 1 or 2 1-(5-(2-quinolinylmethoxy)-2-thienyl)-1-hexanone.

4. Compound of the general formula I according to Claim 1 or 2, alpha-pentyl-5-(2-quinolinylmethoxy-)-2-thiophenmethanol.

5. Process for the preparation of compounds of the general formula I, defined in Claim 1, characterized in that

a) a compound of the formula II

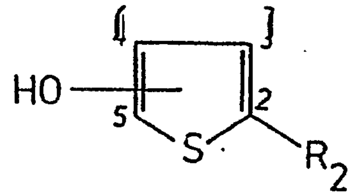

II

in which the hydroxyl group is in position 4 or 5 of the thiophene ring and $R_2$ denotes a $-COO-(C_1-C_4)$-alkyl group or a $-CO-(CH_2)_n-CH_3$ group, wherein n represents an integer from 2 to 6, is reacted with a compound of the formula

$$R-CH_2-Cl \qquad III$$

in which R has the meaning given in the case of formula I, or with the hydrochloride thereof, in the presence of an alkali metal carbonate in a low-boiling polar organic solvent, and, if $R^2$ denotes a $CO-(CH_2)_n-CH_3$ group, if appropriate the product is reduced with a reducing agent, to give a compound of the formula I in which $R^1$ denotes the $-CH(OH)-(CH_2)_n-CH_3$ group, or

b) if $R^1$ in formula I denotes the radical $-CH(OH)-(CH_2)_n-CH_3$ or $-CO-(CH_2)_n-CH_3$, a compound of the formula

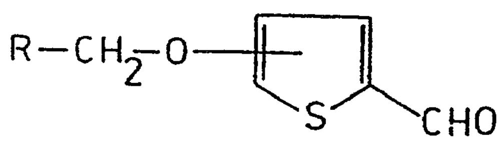

IV

in which R has the meaning given in the case of formula I, is reacted with a compound of the formula

$$CH_3-(CH_2)_n-Y \qquad V$$

in which n denotes an integer from 2 to 6 and Y denotes the Li or MgX group, wherein X represents bromine or iodine, to give a compound of the formula I in which $R_1$ denotes the $-CH(OH)-(CH_2)_n-CH_3$ group, and, if appropriate, the compound thus obtained is oxidized with an oxidizing agent to give a compound of the formula I in which $R_1$ denotes the group $-CO-(CH_2)_n-CH_3$, and

c) if appropriate, compounds of the formula I obtained according to a) or b) are converted into their pharmaceutically acceptable acid addition salts.

6. Process according to Claim 5 for the preparation of compounds of the general formula I in which R1 denotes a $-CH(OH)-(CH_2)_n-CH_3$ group, characterized in that sodium borohydride in ethanol is used for reduction of the compounds of the formula I in which $R_1$ denotes a $-CO-$ (CH2)n–CH3 group.

7. Process according to Claim 5 for the preparation of compounds of the general formula I in which $R_1$ denotes a $-CH(OH)-CH_2)_n-CH_3$ group or $-CO-(CH_2)_n-CH_3$, characterized in that compounds of the formula IV are reacted with lithium-alkyls or Grignard compounds in ether or tetrahydrofuran to give compounds of the formula I on the sheet of formulae in which $R_1$ denotes a $-CH(OH)-(CH_2)_n-CH_3$ group, and, if appropriate, these are oxidized with hexavalent chromium compounds to give compounds of the formula I on the sheet of formulae in which $R_1$ denotes a $-CO-(CH_2)_n-CH_3$ group.

8. Pharmaceutical preparations containing compounds of the general formula I according to Claim 1 in combination with customary galenical auxiliaries and/or excipients.

9. Compounds to Claim 1 for use as active compounds for medicaments for the treatment and prophylaxis of diseases caused by disturbances in arachidonic acid metabolism.

**Claims for the Contracting States: AT, GR, ES**

1. Process for the preparation of new nitrogen-aryl-methoxy-thiophene derivatives of the general formula

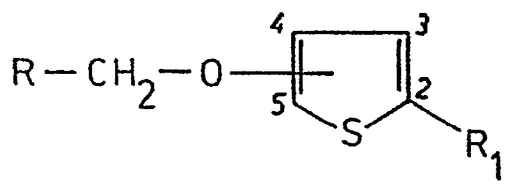

I

in which the R–$CH_2$O-group is in position 4 or 5 of the thiophene ring, R denotes a 2-pyridinyl or 2-quinolinyl group and $R_1$ denotes a –COO–($C_1$–$C_4$)-alkyl, –CO–$(CH_2)_n$–$CH_3$ or CH(OH)–$(CH_2)_n$–$CH_3$ group, wherein n represents an integer from 2 to 6, and their hydrates and/or their pharmaceutically acceptable acid addition salts, characterized in that

a) a compound of the formula II

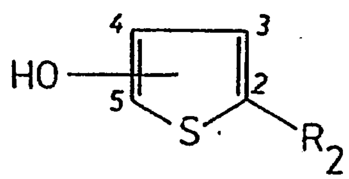

II

in which the hydroxyl group is in position 4 or 5 of the thiophene ring and $R_2$ denotes a –COO–($C_1$–$C_4$)-alkyl group or a –CO–$(CH_2)_n$–$CH_3$ group, wherein n represents an integer from 2 to 6, is reacted with a compound of the formula

R–$CH_2$–Cl III

in which R has the meaning given in the case of formula I, or with the hydrochloride thereof, in the presence of an alkali metal carbonate in a low-boiling polar organic solvent, and, if $R_2$ denotes a –CO–$(CH_2)_n$–$CH_3$ group, if appropriate the product is reduced with a reducing agent, to give a compound of the formula I in which $R_1$ denotes the –CH(OH)–$CH_2)_n$–$CH_3$ group, or

b) if $R_1$ in formula I denotes the radical –CH(OH)–$(CH_3)_n$–$CH_3$ or –CO–$(CH_2)_n$–$CH_3$, a compound of the formula

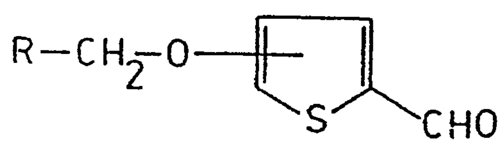

IV

in which R has the meaning given in the case of formula I, is reacted with a compound of the formula

$CH_3$–$(CH_2)_n$–Y V

in which n denotes an integer from 2 to 6 and Y denotes the Li or MgX group, wherein X represents bromine or iodine, to give a compound of the formula I in which $R_1$ denotes the –CH(OH)–$(CH_2)_n$–$CH_3$ group, and, if appropriate, the compound thus obtained is oxidized within an oxidizing agent to give a compound of the formula I in which $R_1$ denotes the group –CO–$(CH_2)_n$–$CH_3$, and

c) if appropriate, compounds of the formula I obtained according to a) or b) are converted into their pharmaceutically acceptable acid addition salts.

2. Process for the preparation of compounds according to Claim 1, in which R, denotes a –CO–$(CH_2)_4$–$CH_3$ or –$COOCH_3$ group, characterized in that compounds of the formula II, in which $R_2$ denotes a –CO–$(CH_2)_4$ or –$COOCH_3$ group, are reacted with compounds of the formula III.

3. Process for the preparation of compounds of the general formula I according to Claim 1 in which $R_1$ denotes a –CH(OH)–$(CH_2)_4$–$CH_3$ group, characterized in that compounds of the formula II, in which $R_2$ denotes a –CO–(CH2)$_4$–$CH_3$ group are reacted with compounds of the formula III and then reduced to compounds of the formula I, in which $R_1$ denotes a –CH(OH)–$(CH_2)_4$–$CH_3$ group.

4. Process according to one of claims 1 or 2, characterized in that 1-(5-hydroxy-2-thienyl)-1-hexanone is reacted with 2-chloromethylquinoline.

5. Process according to claim 1 or 3, characterized in that 1-(5-hydroxy-2-thienyl)-1-hexanone is reacted with 2-chloromethylquinoline and then the keto group is reduced to secondary alcohol.

6. Process according to one of claims 1 to 3 for the preparation of compounds of the general formula 1 in which $R_1$ denotes a –CH(OH)–$(CH_2)_n$–$CH_3$ group, characterized in that the reaction of the compounds

of the formula II is carried out with compounds of the formula III in boiling 2-butanone in the presence of at least 1 mol of anhydrous sodium or potassium carbonate.

7. Process according to Claims 1 to 3 for the preparation of compounds of the general formula I in which $R_1$ denotes a $-CH(OH)-(CH_2)_n-CH_3$ group, characterized in that lithium aluminium hydride or sodium borohydride are used.

8. Process according to Claim 1 for the preparation of compounds of the general formula I in which $R_1$ denotes a $-CH(OH)-CH_2)_n-CH_3$ group or $-CO-(CH_2)_n-CH_3$, characterized in that compounds of the formula IV are reacted with lithium-alkyls or Grignard compounds in ether or tetrahydrofuran to give compounds of the formula I on the sheet of formulae in which $R_1$ denotes a $-CH(OH)-(CH_2)_n-CH_3$ group, and, if appropriate, these are oxidized with hexavalent chromium compounds to give compounds of the formula I on the sheet of formulae in which $R_1$ denotes a $-CO-(CH_2)_n-CH_3$ group.

9. Process for the preparation of pharmaceutical preparations, characterized in that compounds of the general formula I are processed, with pharmaceutical, organic or inorganic auxiliaries and/or excipients which are suitable for oral or parenteral administration, and, if appropriate, other pharmaceutical active compounds, to form solid, semi-solid or liquid medicaments.

**Revendications pour les Etats contractants: BE, CH, FR, GB, IT, LI, LU, NL, SE**

1. Nouveaux dérivés arylméthoxy azotés du thiophène de formule générale:

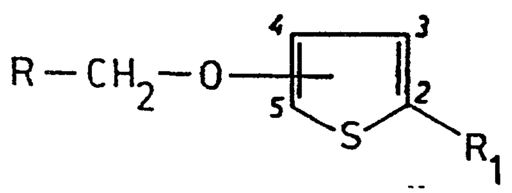

I

dans laquelle le groupe $R-CH_2O$ est placé en position 4 ou 5 du cycle thiophène, R est un groupe 2-pyridinyle ou 2-quinoléinyle et R1 est un groupe $-COO-(C_1-C_4)$alkyle, $-CO-(CH_2)_n-CH_3$ ou $-CH(OH)-(CH_2)_n-CH_3$, où n représente un nombre entier de 2 à 6, ainsi que leurs hydrates et/ou leurs sels d'addition acides pharmaceutiquement admissible.

2. Composés de formule générale (I) selon la revendication 1, dans lesquels $R_1$ est un groupe $-CO-(CH_2)_4-CH_3$, $-CH(OH)-(CH_2)_4-CH_3$ ou $-COOCH_3$.

3. Composé de formule générale (I) selon la revendication 1 ou 2 1-(5-(2-méthoxyquinoléinyl)-2-thiényl)-1-hexanone.

4. Composé de formule générale (I) selon la revendication 1 ou 2, méthanol alpha-pentyl-5-(2-méthoxyquinoléinyl)-2-thiophène.

5. Procédé de préparation des composés de formule générale (I) définis dans la revendication 1, caractérisé en ce que:

(a) on fait réagir un composé de formule générale

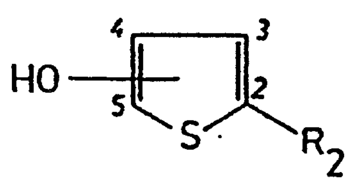

II

dans laquelle le groupe hydroxyle est en position 4 ou 5 du cycle thiophène et $R_2$ représente un groupe $-COO-(C_1-C_4)$alkyle ou un groupe $-CO-(CH_2)_n-CH_3$ où n est un nombre entier compris entre 2 et 6, en présence d'un carbonate alcalin dans un solvant polaire organique à bas point d'ébullition, avec un composé de formule:

$$R-CH_2-Cl \qquad III$$

dans lequel R a la signification indiquée dans la formule (I) ou avec son hydrochlorure, et, dans le cas où $R_2$ signifie le groupe $-CO-(CH_2)_n-CH_3$ on réduit le cas échéant en un composé de formule (I) à l'aide d'un moyen de réduction, dans lequel $R_1$ est un groupe $-CH(OH)-CH_2)_n-CH_3$ ou bien,

(b) si $R_1$ signifie dans la formule (I) un reste $-CH(OH)-(CH_2)_n-CH_3$ ou $-CO-(CH_2)_n-CH_3$, on fait réagir un composé de formule:

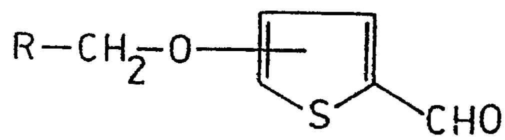

IV

dans laquelle R a la signification indiquée dans la formule (I), avec un composé de formule:

$CH_3-(CH_2)_n-Y$ V

dans laquelle n est un nombre entier compris entre 2 et 6, et Y signifie Li ou MgX, X étant l'iode ou le brome, en un composé de formule (I) dans lequel $R_1$ signifie le groupe $-CH(OH)-(CH_2)_n-CH_3$, et le cas échéant transformé à l'aide d'un moyen d'oxydation le composé ainsi obtenu en un composé de formule (I) où $R_1$ représente le groupe $-CO-(CH_2)n-CH_3$,
(c) le cas échéant transformé les composés de formule (I) obtenus selon (a) ou (b) en leurs sels acides d'addition pharmaceutiquement admissible.

6. Procédé selon la revendication 5 pour préparer des composés de formule générale (I), dans lesquels $R_1$ signifie un groupe $-CH(OH)-(CH_2)_n-CH_3$, caractérisé en ce qu'on réduit les composés de formule (I) dans lesquels $R_1$ signifie un groupe $-CO-(CH_2)_n-CH_3$ en présence de borohydrure de sodium dans l'éthanol.

7. Procédé selon la revendication 5 pour préparer des composés de formule générale (I) dans lesquels $R_1$ est un groupe $-CH(OH)-(CH_2)_n-CH_3$ ou bien $-CO-(CH_2)_n-CH_3$, caractérisé en ce qu'on fait réagir des composés de formule (IV) avec des lithiumalkyles ou des composés de Grignard dans l'éther ou le trétrahydrofurane pour obtenir les composés de formule générale (I) dans lesquels $R_1$ est un groupe $-CH(OH)-(CH_2)_n-CH_3$ et en ce qu'on oxyde ceux-ci le cas échéant avec des composés du chrome à degré d'oxydation 6 en composés de formule (I) selon la page de formule dans lesquels $R_1$ est le groupe $-CO-(CH_2)_n-CH_3$.

8. Préparation pharmaceutique renfermant des composés de formule générale (I) selon la revendication 1, en combinaison avec les substances vectrices ou adjuvantes classiques.

9. Composés selon la revendication 1 utilisés comme principe actif pour médicaments appliqués au traitement et à la prophylaxie des maladies provenant des déréglements des modifications des substances par l'acide arachidique.

**Revendications pour les Etats contractants: AT, GR, ES**

1. Procédé de préparation de nouveau dérivés arylméthoxy azotés du thiophène de formule générale:

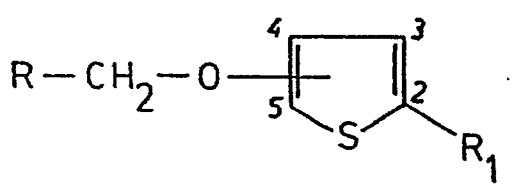

I

dans laquelle le groupe $R-CH_2O$ est placé en position 4 ou 5 du cycle thiophène, R est un groupe 2-pyridinyle ou 2-quinoléinyle et $R_1$ est un groupe $-COO-(C_1-C_4)$alkyle, $-CO(CH_2)_n-CH_3$ ou $-CH(OH)-(CH_2)_n-CH_3$, où n représente un nombre entier de 2 à 6,
ainsi que leurs hydrates et/ou leurs sels d'addition acides pharmaceutiquement admissible, caractérisé en ce que:
(a) on fait réagir un composé de formule générale

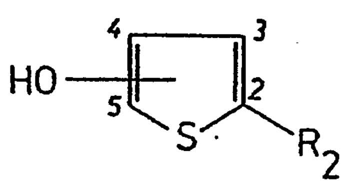

II

dans laquelle le groupe hydroxyle est en position 4 ou 5 du cycle thiophène et $R_2$ représente un groupe $-COO-(C_1-C_4)$alkyle ou un groupe $-CO-(CH_2)_n-CH_3$ où n est un nombre entier compris entre 2 et 6, en présence d'un carbonate alcalin dans un solvant polaire organique à bas point d'ébullition, avec

un composé de formule:

$$R-CH_2-Cl \qquad III$$

dans lequel R a la signification indiquée dans la formule (I) ou avec son hydrochlorure, et, dans le cas où R2 signifie le groupe $-CO-(CH_2)_n-CH_3$ on réduit le cas échéant en un composé de formule (I) à l'aide d'un moyen de réduction, dans lequel $R_1$ est un groupe $-CH(OH)-(CH_2)_n-CH_3$ ou bien,
(b) si $R_1$ signifie dans la formule (I) un reste $-CH(OH)-(CH_2)_n-CH_3$ ou $-CO-(CH_2)_n-CH_3$, on fait réagir un composé de formule:

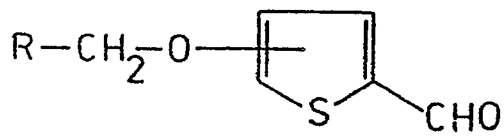

IV

dans laquelle R a la signification indiquée dans la formule (I), avec un composé de formule:

$$CH_3-(CH_2)_n-Y \qquad V$$

dans laquelle n est un nombre entier compris entre 2 et 6, et Y signifie Li ou MgX, X étant l'iode ou le brome, en un composé de formule (I) dans lequel $R_1$ signifie le groupe $-CH(OH)-(CH_2)_n-CH_3$, et le cas échéant transformé à l'aide d'un moyen d'oxydation le composé ainsi obtenu en un composé de formule (I) où $R_1$ représente le groupe $-CO-(CH_2)_n-CH_3$,
(c) le cas échéant transformé les composés de formule (I) obtenus selon (a) ou (b) en leurs sels acides d'addition pharmaceutiquement admissible.

2. Procédé de préparation des composés de formule générale (I) selon la revendication 1, où $R_1$ représente un groupe $-CO-(CH_2)_4-CH_3$ ou $-COOCH_3$, caractérisé en ce qu'on fait réagir les composés de formule (II) dans lesquels $R_2$ est un groupe $-CO(CH_2)_4-CH_3$ ou $-COOCH_3$ avec des composés de formule (III).

3. Procédé de préparation de composés de formule générale (I) selon la revendication 1, dans lesquels $R_1$ représente le groupe $-CH(OH)-(CH_2)_4-CH_3$, caractérisé en ce qu'on fait réagir des composés de formule (II) dans lesquels $R_2$ représente le groupe $-CO-(CH_2)_4-CH_3$ avec des composés de formule (III) que l'on réduit finalement en composés de formule (I) dans lesquels $R_1$ est le groupe $-CH(OH)-CH_2)_4-CH_3$.

4. Procédé selon l'une des revendications 1 ou 2, caractérisé en ce qu'on fait réagir le 1-(5-hydroxy-2-thiényl)-1-hexanone avec la quinoléine-2-chlorométhyle.

5. Procédé selon la revendication 1 ou 3, caractérisé en ce qu'on fait réagir le 1-(5-hydroxy-2-thiényl)-1-hexanone avec la quinoléine 2-chlorométhyle et qu'on réduit finalement le groupe cétone en alcool secondaire.

6. Procédé selon l'une des revendications 1 à 3, pour préparer des composés de formule générale (I) dans lesquels $R_1$ est un groupe $-CH(OH)-(CH_2)_n-CH_3$, caractérisé en ce qu'on réalise la transformation des composés de formule (II) en présence de composés de formule (III) dans le butanone-2 bouillant en présence d'au moins 1 mole de carbonate anhydre de sodium ou de potassium.

7. Procédé selon la revendication 1 pour préparer des composés de formule générale (I) dans lesquels $R_1$ est un groupe $-CH(OH)-(CH_2)_n-CH_3$, caractérisé en ce qu'on réduit les composés de formule (I) dans lesquels $R_1$ est un groupe $-CO-(CH_2)_n-CH_3$ en présence d'hydrure aluminium-lithium ou de borohydrure de sodium.

8. Procédé selon la revendication 1 pour préparer des composés de formule générale (I), dans lesquels $R_1$ est un groupe $-CH(OH)-(CH_2)_n-CH_3$ ou $-CO-(CH_2)_n-CH_3$, caractérisé en ce qu'on fait réagir des composés de formule (IV) avec des alkyles lithium ou des composés de Grignard dans l'éther ou le tétrahydrofurane en composés de formule (I) selon la page de formules dans lesquels $R_1$ est un groupe $-CH(OH)-(CH_2)_n-CH_3$ et en ce qu'on oxyde ceux-ci le cas échéant avec des composés du chrome à degré d'oxydation 6 en composés de formule (I) selon la page de formules dans lesquels $R_1$ est le groupe $-CO-(CH_2)_n-CH_3$.

9. Procédé de préparation de compositions pharmaceutiques, caractérisé en ce qu'on utilise les composés de formule générale (I) avec des substances vetrices et/ou des adjuvants organiques ou non pharmaceutiques aptes aux applications orales ou parentérales et le cas échéant avec d'autres substances pharmaceutiques pour préparer des médicaments solides, semi-durs ou liquides.